(19) Europäisches Patentamt
European Patent Office
Office européen des brevets

(11) EP 4 328 220 A1

(12) EUROPEAN PATENT APPLICATION

(43) Date of publication:
28.02.2024 Bulletin 2024/09

(21) Application number: 23187725.9

(22) Date of filing: 26.07.2023

(51) International Patent Classification (IPC):
*C07D 249/08* (2006.01) *C07D 403/02* (2006.01)
*H01M 10/0566* (2010.01)

(52) Cooperative Patent Classification (CPC):
C07D 249/08; H01M 10/0566

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 22.08.2022 KR 20220104752
29.06.2023 KR 20230084415

(71) Applicant: Samsung SDI Co., Ltd.
Yongin-si, Gyeonggi-do 17084 (KR)

(72) Inventors:
- Park, Hongryeol
  17084 Yongin-si, Gyeonggi-do (KR)
- Choi, Hyunbon
  17084 Yongin-si, Gyeonggi-do (KR)
- Park, Sangwoo
  17084 Yongin-si, Gyeonggi-do (KR)
- Kim, Dahyun
  17084 Yongin-si, Gyeonggi-do (KR)
- Kim, Sundae
  17084 Yongin-si, Gyeonggi-do (KR)
- Yang, Yeji
  17084 Yongin-si, Gyeonggi-do (KR)
- Tsay, Olga
  17084 Yongin-si, Gyeonggi-do (KR)
- Shatunov, Pavel
  17084 Yongin-si, Gyeonggi-do (KR)
- Kim, Sanghoon
  17084 Yongin-si, Gyeonggi-do (KR)

(74) Representative: Gulde & Partner
Patent- und Rechtsanwaltskanzlei mbB
Wallstraße 58/59
10179 Berlin (DE)

# (54) ADDITIVE FOR ELECTROLYTE, AND ELECTROLYTE FOR RECHARGEABLE LITHIUM BATTERY AND RECHARGEABLE LITHIUM BATTERY INCLUDING THE SAME

(57) Provided are an additive for an electrolyte represented by Chemical Formula 1 and an electrolyte for a rechargeable lithium battery and rechargeable lithium battery including the same.

Details of the above Chemical Formula 1 are as described in the specification.

【FIG. 1】

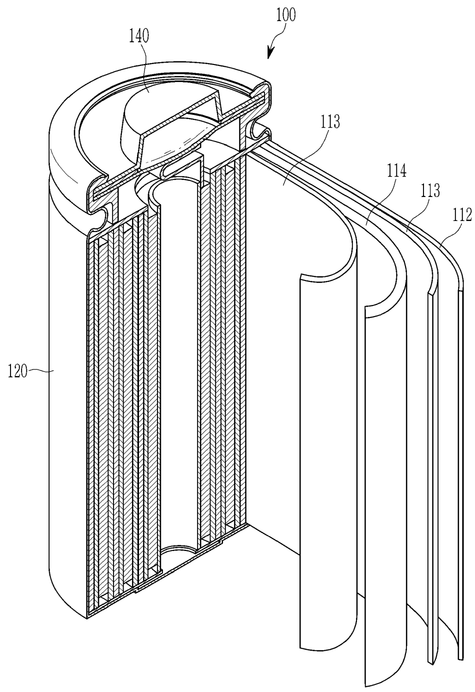


EP 4 328 220 A1

## Description

### BACKGROUND OF THE INVENTION

(a) Field of the Invention

This disclosure relates to an additive for an electrolyte, and an electrolyte for a rechargeable lithium battery and a rechargeable lithium battery including the same.

(b) Description of the Related Art

A rechargeable lithium battery may be recharged and has three or more times as high energy density per unit weight as a conventional lead storage battery, nickel-cadmium battery, nickel hydrogen battery, nickel zinc battery and the like. It may be also charged at a high rate and thus, is commercially manufactured for a laptop, a cell phone, an electric tool, an electric bike, and the like, and researches on improvement of additional energy density have been actively made.

Such a rechargeable lithium battery is manufactured by injecting an electrolyte into a battery cell, which includes a positive electrode including a positive active material capable of intercalating/deintercalating lithium ions and a negative electrode including a negative active material capable of intercalating/deintercalating lithium ions.

The electrolyte serves as a medium for moving lithium ions between the negative electrode and the positive electrode, and in general, an organic solvent in which a lithium salt is dissolved is used, and this electrolyte is important in determining the stability and performance of a rechargeable lithium battery.

The electrolyte may include, for example, a mixed solvent of a high dielectric cyclic carbonate such as propylene carbonate and ethylene carbonate and a chain carbonate such as diethyl carbonate, ethylmethyl carbonate, dimethyl carbonate to which a lithium salt of $LiPF_6$, $LiBF_4$, LiFSI, and the like is added. As the development of batteries in various fields is activated, the development of batteries with high output and high stability in a wide temperature range is becoming more important. In terms of electrolyte, it is important to develop an optimal combination of an organic solvent and an additive capable of improving high output, long cycle-life, and high-temperature storage, and suppressing swelling, capacity reduction, and resistance increase.

### SUMMARY OF THE INVENTION

The invention is defined by the appended claims.

An embodiment provides an additive for an electrolyte having excellent room temperature characteristics and high temperature characteristics.

Another embodiment provides an electrolyte for a rechargeable lithium battery including the additive.

Another embodiment provides a rechargeable lithium battery including the electrolyte.

Provided is a rechargeable lithium battery having improved cycle-life characteristics at room temperature and high temperature, and excellent effect of suppressing an increase in resistance of the battery when left at a high temperature and suppressing gas generation.

### BRIEF DESCRIPTION OF THE DRAWINGS

FIG. 1 is a schematic view of a rechargeable lithium battery according to an embodiment of the present invention.

### DETAILED DESCRIPTION OF THE EMBODIMENTS

Hereinafter, embodiments of the present invention are described in detail. However, these embodiments are exemplary, the present invention is not limited thereto and the present invention is defined by the scope of claims.

As used herein, when a definition is not otherwise provided, "substituted" refers to replacement of at least one hydrogen by deuterium, a halogen, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C10 fluoroalkyl group, or a cyano group. In addition, in specific examples of the present disclosure, "substituted" refers to replacement of at least one hydrogen of a substituent or a compound by deuterium, a halogen, a C1 to C20 alkyl group, a C6 to C30 aryl group, a C1 to C10 fluoroalkyl group, or a cyano group. In addition, in specific examples of the present disclosure, "substituted" refers to replacement of at least one hydrogen of a substituent or a compound by deuterium, a halogen, a C1 to C5 alkyl group, a C6 to C18 aryl group, a C1 to C5 fluoroalkyl group, or a cyano group. In addition, in specific examples of the present disclosure, "substituted" refers to replacement of at least one hydrogen

of a substituent or a compound by deuterium, a cyano group, a halogen, a methyl group, an ethyl group, a propyl group, a butyl group, a phenyl group, a biphenyl group, a terphenyl group, a trifluoromethyl group, or a naphthyl group.

A rechargeable lithium battery may be classified into a lithium ion battery, a lithium ion polymer battery, and a lithium polymer battery depending on kinds of a separator and an electrolyte. It also may be classified to be cylindrical, prismatic, coin-type, pouch-type, and the like depending on shapes. In addition, it may be bulk type and thin film type depending on sizes. Structures and manufacturing methods for lithium ion batteries pertaining to this disclosure are well known in the art.

Herein, a cylindrical rechargeable lithium battery will be exemplarily described as an example of the rechargeable lithium battery. FIG. 1 schematically shows the structure of a rechargeable lithium battery according to an embodiment. Referring to FIG. 1, a rechargeable lithium battery 100 according to an embodiment includes a battery cell including a positive electrode 114, a negative electrode 112 facing the positive electrode 114, a separator 113 between the positive electrode 114 and the negative electrode 112, and an electrolyte (not shown) impregnating the positive electrode 114, the negative electrode 112, and the separator 113, a battery case 120 housing the battery cell, and a sealing member 140 sealing the battery case 120.

Hereinafter, an additive for an electrolyte according to the invention will be described.

The additive according to the present invention is represented by Chemical Formula 1.

[Chemical Formula 1]

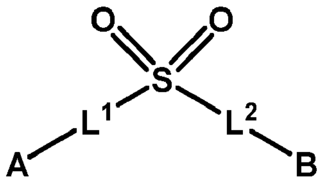

In Chemical Formula 1,

$L^1$ and $L^2$ are each independently a single bond, a substituted or unsubstituted C1 to C5 alkylene group, a substituted or unsubstituted C2 to C5 alkenylene group, a substituted or unsubstituted C2 to C5 alkynylene group, or a substituted or unsubstituted C6 to C20 arylene group,

A and B are each independently a substituted or unsubstituted C1 to C20 alkyl group, a substituted or unsubstituted C6 to C20 aryl group, or a substituted or unsubstituted C2 to C20 heteroaryl group, and

at least one of A and B is a group represented by Chemical Formula A,

[Chemical Formula A]

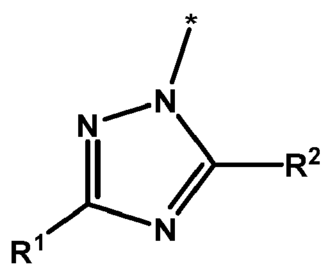

wherein, in Chemical Formula A,
$R^1$ and $R^2$ are each independently hydrogen, a halogen, a substituted or unsubstituted C1 to C10 alkyl group, or a substituted or unsubstituted C3 to C10 cycloalkyl group.

In a rechargeable lithium battery, a non-aqueous electrolyte is decomposed during the initial charge and discharge and thus forms a film having passivation ability on the surfaces of positive and negative electrodes, thereby improving storage characteristics at a high temperature but may be deteriorated by acid such as HF and $PF_5$ produced by thermal decomposition of lithium salts ($LiPF_6$ and the like) which is widely used in lithium batteries. This acid attack

elutes transition metal from the positive electrode and thus increases sheet resistance of the electrode due to structural changes of the surface but reduces theoretical capacity due to the loss of the metal elements, which are redox centers, thereby deteriorating expression capacity. In addition, these eluted transition metal ions are electrodeposited on the negative electrode reacting in a strong reduction potential band and thus may not only consume electrons but also destroy the film during the electrodeposition and expose the surface of the negative electrode, thereby additionally causing the decomposition reaction of an electrolyte. As a result, as the resistance of the negative electrode increases, and irreversible capacity increases, there is a problem of continuously deteriorating capacity of a cell. In the present invention, a triazole group and a sulfone group of the additive represented by Chemical Formula 1 provide a lone pair of electrons to capture $PF_5$ and stabilize a $LiPF_6$ salt, thereby removing the acid caused due to the decomposition of the lithium salt.

In addition, the sulfone group included in Chemical Formula 1 forms a film on the surface of the positive electrode to suppress decomposition of the positive active material, thereby suppressing gas generation and elution of transition metals due to the decomposition of the positive electrode material.

In addition, the aforementioned additive represented by Chemical Formula 1 may strengthen the SEI (Solid Electrolyte Interface) film on the surface of the positive electrode while preventing deterioration of the SEI film or elution of transition metals from the positive electrode during high-temperature storage.

For example, at least one of $L^1$ and $L^2$ may be a substituted or unsubstituted C1 to C5 alkylene group.

For example, $L^1$ and $L^2$ may each independently be a substituted or unsubstituted C1 to C5 alkylene group.

For example, at least one of $L^1$ and $L^2$ may be a substituted or unsubstituted C2 to C5 alkylene group.

For example, $L^1$ and $L^2$ may each independently be a substituted or unsubstituted C2 to C5 alkylene group.

According to an embodiment, $L^1$ and $L^2$ are identical. Symmetric compounds falling under Formula 1 can usually be achieved with less synthetic effort. In particular, $L^1$ and $L^2$ may be both a substituted or unsubstituted C1 to C5 alkylene group.

For example, Chemical Formula 1 may be represented by Chemical Formula 1-1.

[Chemical Formula 1-1]

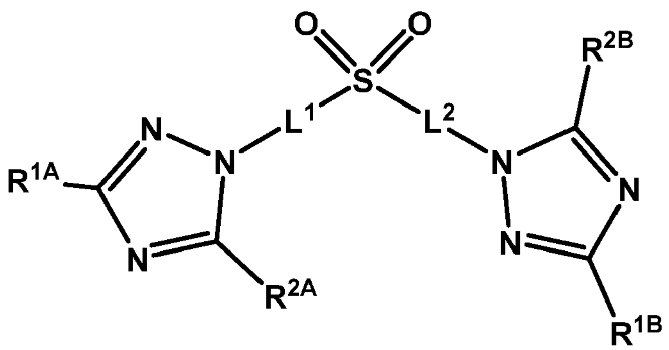

In Chemical Formula 1-1,

$L^1$ and $L^2$ are each independently a substituted or unsubstituted C1 to C5 alkylene group, in particular a substituted or unsubstituted C2 to C5 alkylene group, most preferred a substituted or unsubstituted C2 or C3 alkylene group, and

$R^{1A}$, $R^{1B}$, $R^{2A}$, and $R^{2B}$ are each independently hydrogen, a halogen, a substituted or unsubstituted C1 to C10 alkyl group, or a substituted or unsubstituted C3 to C10 cycloalkyl group. Preferably, $L^1$ and $L^2$ are identical and/or $R^{1A}$, $R^{1B}$, $R^{2A}$, and $R^{2B}$ are each hydrogen.

In an embodiment, the additive for the electrolyte may be selected from the compounds of Formula 1a or Formula 1b.

[Formula 1a] 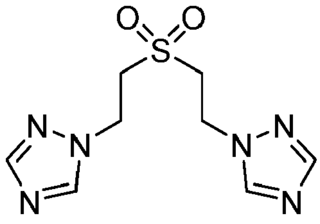

[Formula 1b] 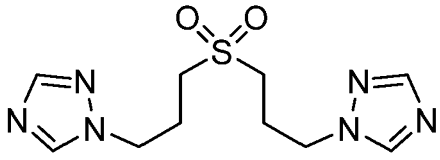 .

The electrolyte for a rechargeable lithium battery according to another embodiment includes a non-aqueous organic solvent, a lithium salt, and the aforementioned additive for the electrolyte.

The additive may be included in an amount of about 0.01 to 5.0 parts by weight based on 100 parts by weight of the electrolyte for the rechargeable lithium battery.

For example, the additive may be included in an amount of about 0.01 to 4.0 parts by weight, about 0.01 to 3.0 parts by weight, about 0.03 to 3.0 parts by weight, or about 0.05 to 3.0 parts by weight based on 100 parts by weight of the electrolyte for a rechargeable lithium battery.

When the content range of the additive is as described above, it is possible to implement a rechargeable lithium battery having improved cycle-life characteristics and output characteristics by preventing an increase in resistance at high temperatures.

The electrolyte for the rechargeable lithium battery may further include at least one other additive of vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene carbonate, vinylethylene carbonate (VEC), adiponitrile (AN), succinonitrile (SN), 1,3,6-hexane tricyanide (HTCN), propenesultone (PST), propanesultone (PS), lithium tetrafluoroborate ($LiBF_4$), lithium difluorophosphate ($LiPO_2F_2$), and 2-fluoro biphenyl (2-FBP).

By further including the aforementioned other additives, the cycle-life may be further improved or gases generated from the positive electrode and the negative electrode may be effectively controlled during high-temperature storage.

The other additives may be included in an amount of about 0.2 to 20 parts by weight, specifically about 0.2 to 15 parts by weight, for example, about 0.2 to 10 parts by weight, based on 100 parts by weight of the electrolyte for the rechargeable lithium battery.

When the content of other additives is as described above, an increase in film resistance may be minimized, thereby contributing to the improvement of battery performance.

The non-aqueous organic solvent serves as a medium for transmitting ions taking part in the electrochemical reaction of a battery.

The non-aqueous organic solvent may be a carbonate-based, ester-based, ether-based, ketone-based, alcohol-based, or aprotic solvent.

The carbonate-based solvent may include dimethyl carbonate (DMC), diethyl carbonate (DEC), dipropyl carbonate (DPC), methylpropyl carbonate (MPC), ethylpropyl carbonate (EPC), ethylmethyl carbonate (EMC), ethylene carbonate (EC), propylene carbonate (PC), butylene carbonate (BC), and the like. The ester-based solvent may include methyl acetate, ethyl acetate, n-propyl acetate, t-butyl acetate, methylpropionate, ethylpropionate, propylpropionate, decanolide, mevalonolactone, caprolactone, and the like. The ether-based solvent may include dibutyl ether, tetraglyme, diglyme, dimethoxyethane, 2-methyltetrahydrofuran, tetrahydrofuran, and the like. In addition, the ketone-based solvent may include cyclohexanone, and the like. The alcohol-based solvent may include ethanol, isopropyl alcohol, and the like and the aprotic solvent may include nitriles such as $R^1$-CN (wherein $R^1$ is a hydrocarbon group having a C2 to C20 linear, branched, or cyclic structure and may include a double bond, an aromatic ring, or an ether bond), and the like, dioxolanes such as 1,3-dioxolane, and the like, sulfolanes, and the like.

The non-aqueous organic solvent may be used alone or in a mixture. When the organic solvent is used in a mixture, the mixture ratio may be controlled in accordance with a desirable battery performance.

The carbonate-based solvent is prepared by mixing a cyclic carbonate and a chain carbonate. When the cyclic carbonate and chain carbonate are mixed together in a volume ratio of about 1:1 to about 9:1, an electrolyte performance

may be improved.

In particular, in an embodiment of the present disclosure, the non-aqueous organic solvent may include the cyclic carbonate and the chain carbonate in a volume ratio of about 2:8 to about 5:5, and as a specific example, the cyclic carbonate and the chain carbonate may be included in a volume ratio of about 2:8 to about 4:6.

As a more specific example, the cyclic carbonate and the chain carbonate may be included in a volume ratio of about 2:8 to about 3:7.

The non-aqueous organic solvent may further include an aromatic hydrocarbon-based organic solvent in addition to the carbonate-based solvent. Herein, the carbonate-based solvent and the aromatic hydrocarbon-based organic solvent may be mixed in a volume ratio of about 1:1 to about 30:1.

The aromatic hydrocarbon-based organic solvent may be an aromatic hydrocarbon-based compound of Chemical Formula 3.

[Chemical Formula 3]

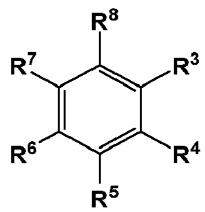

In Chemical Formula 3, $R^3$ to $R^8$ are the same or different and are selected from hydrogen, a halogen, a C1 to C10 alkyl group, a C1 to C10 haloalkyl group, and a combination thereof.

Specific examples of the aromatic hydrocarbon-based organic solvent may be selected from benzene, fluorobenzene, 1,2-difluorobenzene, 1,3-difluorobenzene, 1,4-difluorobenzene, 1,2,3-trifluorobenzene, 1,2,4-trifluorobenzene, chlorobenzene, 1,2-dichlorobenzene, 1,3-dichlorobenzene, 1,4-dichlorobenzene, 1,2,3-trichlorobenzene, 1,2,4-trichlorobenzene, iodobenzene, 1,2-diiodobenzene, 1,3-diiodobenzene, 1,4-diiodobenzene, 1,2,3-triiodobenzene, 1,2,4-triiodobenzene, toluene, fluorotoluene, 2,3-difluorotoluene, 2,4-difluorotoluene, 2,5-difluorotoluene, 2,3,4-trifluorotoluene, 2,3,5-trifluorotoluene, chlorotoluene, 2,3-dichlorotoluene, 2,4-dichlorotoluene, 2,5-dichlorotoluene, 2,3,4-trichlorotoluene, 2,3,5-trichlorotoluene, iodotoluene, 2,3-diiodotoluene, 2,4-diiodotoluene, 2,5-diiodotoluene, 2,3,4-triiodotoluene, 2,3,5-triiodotoluene, xylene, and a combination thereof.

The lithium salt dissolved in the non-aqueous organic solvent supplies lithium ions in a battery, enables a basic operation of a rechargeable lithium battery, and improves transportation of the lithium ions between positive and negative electrodes. Examples of the lithium salt include at least one selected from $LiPF_6$, $LiBF_4$, $LiSbF_6$, $LiAsF_6$, $Li(FSO_2)_2N$ (lithium bis(fluorosulfonyl)imide: LiFSI), $LiC_4F_9SO_3$, $LiClO_4$, $LiAlO_2$, $LiAlCl_4$, $LiPO_2F_2$, $LiN(C_xF_{2x+1}SO_2)(C_yF_{2y+1}SO_2)$, wherein, x and y are an integer ranging from 1 to 20), LiCl, LiI, $LiB(C_2O_4)_2$ (lithium bis(oxalato) borate: LiBOB), LiDFOB (lithium difluoro(oxalato)borate), and $Li[PF_2(C_2O_4)_2]$ (lithium difluoro(bisoxalato) phosphate). The lithium salt may be used in a concentration ranging from about 0.1 M to about 2.0 M. When the lithium salt is included at the above concentration range, an electrolyte may have excellent performance and lithium ion mobility due to optimal electrolyte conductivity and viscosity.

Another embodiment provides a rechargeable lithium battery including a positive electrode including a positive active material; a negative electrode including a negative active material; and the aforementioned electrolyte.

The positive electrode includes a positive electrode current collector and a positive active material layer on the positive electrode current collector, and the positive active material layer includes a positive active material.

The positive active material may include lithiated intercalation compounds that reversibly intercalate and deintercalate lithium ions.

Specifically, at least one composite oxide of a metal selected from cobalt, manganese, nickel, iron, and a combination thereof, and lithium may be used.

Of course, the composite oxide in which a portion of the metal is substituted with other metal may be used, and a phosphoric acid compound of the composite oxide, for example, at least one selected from $LiNiPO_4$, $LiFePO_4$, $LiCoPO_4$, and $LiMnPO_4$, may be used. One having a coating layer on the surface of the composite oxide may be used, or a mixture of the composite oxide and the composite oxide having a coating layer may be used. The coating layer may include at least one coating element compound selected from an oxide of a coating element, a hydroxide of a coating element, an oxyhydroxide of a coating element, an oxycarbonate of a coating element, and a hydroxy carbonate of a coating element. The compound for the coating layer may be amorphous or crystalline. The coating element included in the coating layer may include Mg, Al, Co, K, Na, Ca, Si, Ti, V, Sn, Ge, Ga, B, As, Zr, or a mixture thereof. The coating

layer may be disposed in a method having no adverse influence on properties of a positive active material by using these elements in the coating. For example, the method may include any coating method (e.g., spray coating, dipping, etc.), but is not illustrated in more detail since it is well-known to those skilled in the related field.

The positive active material may be, for example, at least one of lithium composite oxides represented by Chemical Formula 4.

[Chemical Formula 4] $Li_xM^1_yM^2_zM^3_{1-y-z}O_{2\pm a}X_a$

In Chemical Formula 4,
$0.5\leq x\leq 1.8$, $0\leq a\leq 0.1$, $0<y\leq 1$, $0\leq z\leq 1$, $0<y+z\leq 1$, $M^1$, $M^2$, and $M^3$ are each independently one or more elements selected from metals of Ni, Co, Mn, Al, B, Ba, Ca, Ce, Cr, Fe, Mo, Nb, Si, Sr, Mg, Ti, V, W, Zr, or La, and a combination thereof, and X includes one or more elements selected from F, S, P or Cl.

In an embodiment, the positive active material may be at least one selected from $LiCoO_2$, $LiNiO_2$, $LiMnO_2$, $LiMn_2O_4$, $LiNi_aMn_bCo_cO_2$ (a+b+c=1), $LiNi_aMn_bCo_cAl_dO_2$ (a+b+c+d=1), and $LiNi_eCo_fAl_gO_2$ (e+f+g=1).

In Chemical Formula 4, $0.8\leq y\leq 1$, $0\leq z\leq 0.2$, and $M^1$ may be Ni.

For example, the positive active material selected from $LiNi_bMn_cCo_dO_2$ (b+c+d=1), $LiNi_bMn_cCo_dAl_eO_2$ (b+c+d+e=1), and $LiNi_bCo_dAl_eO_2$ (b+d+e=1) may be a high Ni-based positive active material.

For example, in the case of $LiNi_bMn_cCo_dO_2$ (b+c+d=1) and $LiNi_bMn_cCo_dAl_eO_2$ (b+c+d+e=1), a nickel content may be greater than or equal to about 60% ($b \geq 0.6$), and more specifically, greater than or equal to about 80% ($b \geq 0.8$).

For example, in the case of $LiNi_bCo_dAl_eO_2$ (b+d+e=1), a nickel content may be greater than or equal to about 60% ($b \geq 0.6$), and more specifically, greater than or equal to about 80% ($b \geq 0.8$).

The positive active material may be included in an amount of about 90 wt% to about 98 wt% based on the total weight of the positive active material layer.

The conductive material and the binder may be included in an amount of about 1 wt% to about 5 wt% based on the total weight of the positive active material layer.

The conductive material is included to provide electrode conductivity. Any electrically conductive material may be used as a conductive material unless it causes a chemical change. Examples of the conductive material may include a carbon-based material such as natural graphite, artificial graphite, carbon black, acetylene black, ketjen black, a carbon fiber, and the like; a metal-based material of a metal powder or a metal fiber including copper, nickel, aluminum, silver, and the like; a conductive polymer such as a polyphenylene derivative; or a mixture thereof.

The binder improves binding properties of positive active material particles with one another and with a current collector. Examples thereof may be polyvinyl alcohol, carboxylmethyl cellulose, hydroxypropyl cellulose, diacetyl cellulose, polyvinylchloride, carboxylated polyvinylchloride, polyvinylfluoride, an ethylene oxide-containing polymer, polyvinylpyrrolidone, polyurethane, polytetrafluoroethylene, polyvinylidene fluoride, polyethylene, polypropylene, a styrene-butadiene rubber, an acrylated styrene-butadiene rubber, an epoxy resin, nylon, and the like, but are not limited thereto.

The positive electrode current collector may use Al, but is not limited thereto.

The negative electrode includes a negative electrode current collector and a negative active material layer including a negative active material formed on the negative electrode current collector.

The negative active material may include a material that reversibly intercalates/deintercalates lithium ions, a lithium metal, a lithium metal alloy, a material being capable of doping/dedoping lithium, or transition metal oxide.

The material that reversibly intercalates/deintercalates lithium ions may include a carbon material. The carbon material may be any generally-used carbon-based negative active material in a rechargeable lithium battery. Examples thereof may be crystalline carbon, amorphous carbon, or a mixture thereof. The crystalline carbon may be non-shaped, or sheet, flake, spherical, or fiber shaped natural graphite or artificial graphite. The amorphous carbon may be a soft carbon, a hard carbon, a mesophase pitch carbonization product, calcined coke, and the like.

The lithium metal alloy includes an alloy of lithium and a metal selected from Na, K, Rb, Cs, Fr, Be, Mg, Ca, Sr, Si, Sb, Pb, In, Zn, Ba, Ra, Ge, Al, and Sn.

The material being capable of doping/dedoping lithium may be Si, Si-C composite, $SiO_x$ ($0 < x < 2$), a Si-Q alloy wherein Q is an element selected from an alkali metal, an alkaline-earth metal, a Group 13 element, a Group 14 element except Si, a Group 15 element, a Group 16 element, a transition metal, a rare earth element, and a combination thereof), Sn, $SnO_2$, a Sn-$R^{11}$ alloy (wherein $R^{11}$ is an element selected from an alkali metal, an alkaline-earth metal, a Group 13 element, a Group 14 element except Sn, a Group 15 element, a Group 16 element, a transition metal, a rare earth element, and a combination thereof), and the like. At least one of these materials may be mixed with $SiO_2$.

The elements Q and $R^{11}$ may be selected from Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Tl, Ge, P, As, Sb, Bi, S, Se, Te, Po, and a combination thereof.

The transition metal oxide may be vanadium oxide, lithium vanadium oxide, or lithium titanium oxide.

In a specific embodiment, the negative active material may include at least one of graphite and a Si composite.

The Si composite may include a core including Si-based particles and an amorphous carbon coating layer, and for example, the Si-based particles may include at least one of Si particles, a Si-C composite, $SiO_x$ ($0 < x \leq 2$), and a Si alloy.

For example, the Si composite may include a void in the center of the core including the Si-based particles, a radius of the center corresponds to 30% to 50% of the radius of the Si composite, an average particle diameter of the Si composite may be about 5 μm to 20μm and an average particle diameter of the Si-based particles may be about 10 nm to about 200 nm.

In the present specification, the average particle diameter may be a particle size (D50) at 50% by volume in a cumulative size-distribution curve.

When the average particle diameter of the Si-based particle is within the above range, volume expansion occurring during charging and discharging may be suppressed, and a break in a conductive path due to particle crushing during charging and discharging may be prevented.

The core including the Si-based particles further includes amorphous carbon, and in this case, the central portion does not include amorphous carbon, and the amorphous carbon may exist only on the surface portion of the Si composite.

In this case, the surface portion means a region from the outermost surface of the central portion to the outermost surface of the Si composite.

In addition, the Si-based particles are substantially uniformly included in the Si composite as a whole, that is, Si-based particles may be present in a substantially uniform concentration in the center portion and the surface portion of the Si composite.

The amorphous carbon may be soft carbon, hard carbon, a mesophase pitch carbonized product, calcined coke, or a combination thereof.

For example, the Si-C composite may include Si particles and crystalline carbon.

The Si particles may be included in an amount of about 1 wt% to about 60 wt%, for example, about 3 wt% to about 60 wt%, based on the total weight of the Si-C composite.

The crystalline carbon may be, for example, graphite, and specifically, natural graphite, artificial graphite, or a combination thereof.

The average particle diameter of the crystalline carbon may be about 5 μm to about 30 μm.

When the negative active material includes both graphite and Si composite, the graphite and Si composite may be included in the form of a mixture, and in this case, the graphite and Si composite may be included in a weight ratio of about 99: 1 to about 50: 50.

More specifically, the graphite and Si composite may be included in a weight ratio of about 97: 3 to about 80: 20, or about 95: 5 to about 80: 20.

The amorphous carbon precursor may include a coal-based pitch, mesophase pitch, petroleum-based pitch, coal-based oil, petroleum-based heavy oil, or a polymer resin such as a phenol resin, a furan resin, or a polyimide resin.

In the negative active material layer, the negative active material may be included in an amount of about 95 wt% to about 99 wt% based on the total weight of the negative active material layer.

In an embodiment of the present invention, the negative active material layer includes a binder, and optionally includes a conductive material. In the negative active material layer, a content of the binder may be about 1 wt% to about 5 wt% based on a total weight of the negative active material layer. When the negative active material layer includes a conductive material, the negative active material layer includes about 90 wt% to about 98 wt% of the negative active material, about 1 wt% to about 5 wt% of the binder, and about 1 wt% to about 5 wt% of the conductive material.

The binder improves binding properties of negative active material particles with one another and with a current collector. The binder includes a non-water-soluble binder, a water-soluble binder, or a combination thereof.

The non-water-soluble binder may be selected from polyvinylchloride, carboxylated polyvinylchloride, polyvinylfluoride, polyurethane, polytetrafluoroethylene, polyvinylidene fluoride, polyethylene, polypropylene, polyamideimide, polyimide, or a combination thereof.

The water-soluble binder may be a rubber-based binder or a polymer resin binder. The rubber-based binder may be selected from a styrene-butadiene rubber, an acrylated styrene-butadiene rubber (SBR), an acrylonitrile-butadiene rubber, an acrylic rubber, a butyl rubber, a fluorine rubber, and a combination thereof. The polymer resin binder may be selected from polytetrafluoroethylene, an ethylenepropylene copolymer, polyethyleneoxide, polyvinylpyrrolidone, polyepichlorohydrin, polyphosphazene, polyacrylonitrile, polystyrene, an ethylenepropylenediene copolymer, polyvinylpyridine, chlorosulfonated polyethylene, latex, a polyester resin, an acrylic resin, a phenolic resin, an epoxy resin, polyvinyl alcohol, and a combination thereof.

When the water-soluble binder is used as a negative electrode binder, a cellulose-based compound may be further used to provide viscosity as a thickener. The cellulose-based compound includes one or more of carboxymethyl cellulose, hydroxypropylmethyl cellulose, methyl cellulose, or alkali metal salts thereof. The alkali metal may be Na, K, or Li. Such a thickener may be included in an amount of about 0.1 to about 3 parts by weight based on 100 parts by weight of the negative active material.

The conductive material is included to provide electrode conductivity. Any electrically conductive material may be used as a conductive material unless it causes a chemical change. Examples of the conductive material include a carbon-based material such as natural graphite, artificial graphite, carbon black, acetylene black, ketjen black, a carbon fiber, and the like; a metal-based material of a metal powder or a metal fiber including copper, nickel, aluminum, silver, and the like; a conductive polymer such as a polyphenylene derivative; or a mixture thereof.

The negative electrode current collector may include one selected from a copper foil, a nickel foil, a stainless steel foil, a titanium foil, a nickel foam, a copper foam, a polymer substrate coated with a conductive metal, and a combination thereof.

The rechargeable lithium battery may further include a separator between the negative electrode and the positive electrode, depending on a type of the rechargeable lithium battery. Such a separator is a porous substrate; or a composite porous substrate.

The porous substrate may be a substrate including pores, through which lithium ions can move. The porous substrate may include, for example, polyethylene, polypropylene, polyvinylidene fluoride, or a multilayer film of two or more layers thereof, and a mixed multilayer film such as a polyethylene/polypropylene double-layered separator, a polyethylene/polypropylene/polyethylene triple-layered separator, a polypropylene/polyethylene/polypropylene triple-layered separator, or the like may be used.

The composite porous substrate may have a form including a porous substrate and a functional layer on the porous substrate. The functional layer may be, for example, at least one of a heat-resistant layer and an adhesive layer from the viewpoint of enabling additional functions to be added. For example, the heat-resistant layer may include a heat-resistant resin and optionally a filler.

In addition, the adhesive layer may include an adhesive resin and optionally a filler.

The filler may be an organic filler or an inorganic filler.

Hereinafter, examples of the present invention and comparative examples are described. These examples, however, are not in any sense to be interpreted as limiting the scope of the invention.

(Synthesis Example: Synthesis of Additives)

**Synthesis Example 1: Synthesis of Chemical Formula 1a**

After preparing a solution by mixing 1H-1,2,4-triazole (2 mmol) with acetone and sufficiently stirring the mixture with sodium hydrogen carbonate (3 mmol), divinyl sulfone (1.1 mmol) was mixed with 30 mL of acetone and then, added dropwise to the solution for 30 minutes. Subsequently, the mixture was stirred at room temperature (25 °C) for 4 hours, and precipitates were filtered therefrom. The filtered solution was recrystallized, obtaining a compound of Chemical Formula 1a.

[Chemical Formula 1a]

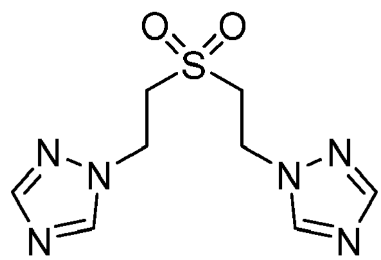

**Synthesis Example 2: Synthesis of Chemical Formula 1b**

A compound of Chemical Formula 1b was obtained in the same manner as in Synthesis Example 1 except that 3-(allylsulfonyl)prop-1-ene was used instead of the divinyl sulfone.

[Chemical Formula 1b]

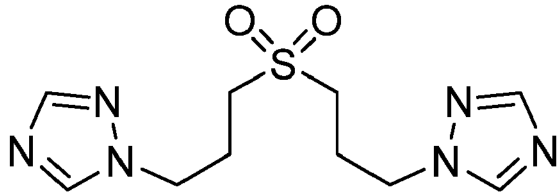

**Synthesis Example 3: Synthesis of Chemical Formula 1c**

1H-1,2,4-triazole was mixed with sodium hydroxide dissolved in water and then, heated at 200 °C for 40 minutes under 8 Pa. Subsequently, sulfuryl fluoride was mixed therewith and then, treated for 18 minutes under 67 kPa, obtaining a compound of Chemical Formula 1c.

[Chemical Formula 1c]

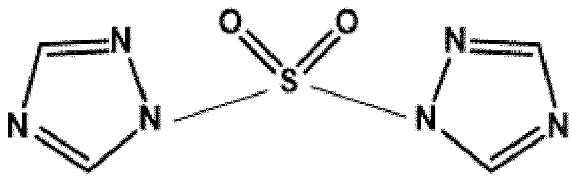

**Comparative Synthesis Example 1: Synthesis of Chemical Formula C3**

A compound of Chemical Formula C3 was obtained in the same manner as in Synthesis Example 1 except that pyrrole was used instead of the 1H-1,2,4-triazole.

[Chemical Formula C3]

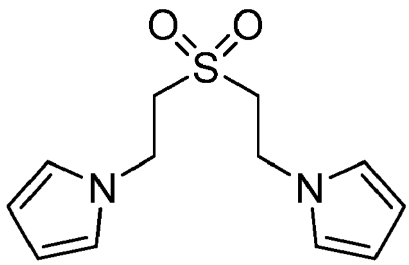

**Comparative Synthesis Example 2: Synthesis of Chemical Formula C4**

A compound of Chemical Formula C4 was obtained in the same manner as in Synthesis Example 3 except that 4H-1,2,4-triazole was used instead of the 1H-1,2,4-triazole.

[Chemical Formula C4]

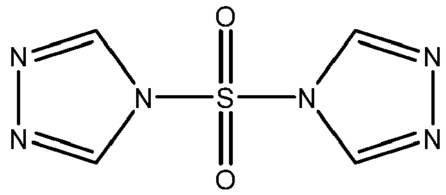

**Comparative Synthesis Example 3: Synthesis of Chemical Formula C5**

A compound of Chemical Formula C5 was obtained in the same manner as in Synthesis Example 3 except that 1H-imidazole was used instead of the 1H-1,2,4-triazole.

[Chemical Formula C5]

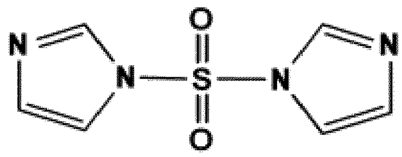

(Example: Manufacture of Rechargeable Lithium Battery Cell)

**Example 1**

Positive active material slurry was prepared by using $LiNi_{0.88}Co_{0.07}Al_{0.05}O_2$ as a positive active material, polyvinylidene fluoride as a binder, and acetylene black as a conductive material in a weight ratio of 96:2:2, and dispersing the mixture in N-methyl pyrrolidone.

The positive active material slurry was coated on a 14 pm-thick Al foil, dried at 110 °C, and pressed to manufacture a positive electrode.

A mixture of artificial graphite and Si composite in a weight ratio of 93:7 as a negative active material, a styrene-butadiene rubber binder as a binder and carboxymethyl cellulose as a thickener were mixed in a weight ratio of 97:1:2, respectively and dispersed in distilled water to prepare a negative active material slurry.

The Si composite has a core including artificial graphite and silicon particles, and a coal-based pitch coated on the surface of the core.

The negative active material slurry was coated on a 10 μm-thick Cu foil, dried at 100°C, and pressed to manufacture a negative electrode.

An electrode assembly was manufactured by assembling the manufactured positive and negative electrodes, and a separator made of polyethylene having a thickness of 25 μm, and an electrolyte was injected to prepare a rechargeable lithium battery cell.

The electrolyte has a following composition.

(Composition of Electrolyte)

Lithium salt: 1.15 M $LiPF_6$

non-aqueous organic solvent: ethylene carbonate: ethylmethyl carbonate: dimethyl carbonate (EC: EMC:DMC = a volume ratio of 20:10:70)

Additive: 0.5 parts by weight of the compound represented by Chemical Formula 1a, 10 parts by weight of fluoroethylene carbonate (FEC), and 0.5 parts by weight of succinonitrile (SN)

(in the composition of the electrolyte, "parts by weight" means a relative weight of the additive based on 100 weight

of the total (lithium salt non-aqueous organic solvent) of the electrolyte excluding the additive.)

**Comparative Example 1**

A rechargeable lithium battery cell was manufactured in the same manner as in Example 1 except that an additive not including the compound of Chemical Formula 1a was used.

**Examples 2 and 3 and Comparative Examples 2 to 6**

Each rechargeable lithium battery cell was manufactured in the same manner as in Example 1 except that the compound of Chemical Formula 1b (Example 2), the compound of Chemical Formula 1c (Example 3), the compound of Chemical Formula C1 (Wako Pure Chemical Industries, Ltd.) (Comparative Example 2), the compound of Chemical Formula C2 (Sigma-Aldrich Co., Ltd.) (Comparative Example 3), and the compounds of Chemical Formulas C3 to C5 (Comparative Examples 4 to 6) were respectively used instead of the compound of Chemical Formula 1a.

[Chemical Formula C1]

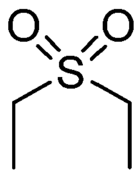

[Chemical Formula C2]

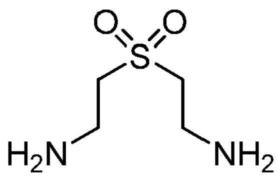

**Examples 4 to 6**

A rechargeable lithium battery cell was manufactured in the same manner as in Example 1 except that the compound of Chemical Formula 1a was used respectively in an amount of 0.1 parts by weight, 2.0 parts by weight, 5.0 parts by weight.

The additives for an electrolyte according to Examples 1 to 6 and Comparative Examples 1 to 6 had a composition shown in Table 1.

(Table 1)

| | Additive (parts by weight) | Other additives | | Solvent (volume ratio) | | |
|---|---|---|---|---|---|---|
| | | FEC (parts by weight) | SN (parts by weight) | EC | EMC | DMC |
| Example 1 | Chemical Formula 1a (0.5) | 10 | 0.5 | 20 | 10 | 70 |
| Example 2 | Chemical Formula 1b (0.5) | 10 | 0.5 | 20 | 10 | 70 |
| Example 3 | Chemical Formula 1c (0.5) | 10 | 0.5 | 20 | 10 | 70 |

(continued)

| | Additive (parts by weight) | Other additives | | Solvent (volume ratio) | | |
|---|---|---|---|---|---|---|
| | | FEC (parts by weight) | SN (parts by weight) | EC | EMC | DMC |
| Example 4 | Chemical Formula 1a (0.1) | 10 | 0.5 | 20 | 10 | 70 |
| Example 5 | Chemical Formula 1a (2.0) | 10 | 0.5 | 20 | 10 | 70 |
| Example 6 | Chemical Formula 1a (5.0) | 10 | 0.5 | 20 | 10 | 70 |
| Comparative Example 1 | - | 10 | 0.5 | 20 | 10 | 70 |
| Comparative Example 2 | Chemical Formula C1 (0.5) | 10 | 0.5 | 20 | 10 | 70 |
| Comparative Example 3 | Chemical Formula C2 (0.5) | 10 | 0.5 | 20 | 10 | 70 |
| Comparative Example 4 | Chemical Formula C3 (0.5) | 10 | 0.5 | 20 | 10 | 70 |
| Comparative Example 5 | Chemical Formula C4 (0.5) | 10 | 0.5 | 20 | 10 | 70 |
| Comparative Example 6 | Chemical Formula C5 (0.5) | 10 | 0.5 | 20 | 10 | 70 |

**Evaluation 1: Evaluation of Room-temperature Charge and Discharge Cycle Characteristics**

The rechargeable lithium battery cells according to Examples 1 to 6 and Comparative Examples 1 to 6 were charged and discharged under the following conditions and evaluated with respect to cycle characteristics, and the results are shown in Table 2.

After 200 cycles performing 0.33 C charges (CC/CV, 4.3 V, 0.025 C Cut-off) /1.0 C discharges (CC, 2.5 V Cut-off) at 25 °C, the cells were evaluated with respect to capacity retention rates and direct current internal resistance (DC-IR) variation ratio.

DC-IR was calculated according to Equations 1 and 2 based on a voltage changed by applying a current of SOC 50 C for 30 seconds, and the results are shown in Table 2.

[Equation 1]

Capacity retention rate = (Capacity after 200 cycles / Capacity after 1 cycle) * 100

[Equation 2]

DC-IR (direct current internal resistance) variation ratio after 200 cycles = {(DC-IR after 200 cycles)/(Initial DC-IR)} * 100

(Table 2)

| | Capacity retention (@25°C, 200 Cycle) | Initial DC-IR @25°C (mΩ) | DC-IR after 200 cycles @25°C (mΩ) | DC-IR variation ratio after 200 cycles @25°C (%) |
|---|---|---|---|---|
| Example 1 | 96.7 | 41.2 | 63.3 | 154 |
| Example 2 | 94.4 | 47.5 | 73.6 | 155 |
| Example 3 | 92.1 | 41.5 | 66.0 | 159 |
| Example 4 | 90.5 | 40.9 | 65.2 | 159 |
| Example 5 | 96.6 | 41.4 | 63.5 | 153 |
| Example 6 | 90.2 | 42.5 | 70.4 | 166 |
| Comparative Example 1 | 53.7 | 42.0 | 147.2 | 350 |
| Comparative Example 2 | 61.8 | 43.8 | 74.5 | 170 |
| Comparative Example 3 | 81.6 | 44.7 | 85.7 | 192 |
| Comparative Example 4 | 86.6 | 43.3 | 74.9 | 173 |
| Comparative Example 5 | 80.8 | 43.5 | 79.2 | 182 |
| Comparative Example 6 | 79.4 | 45.3 | 83.8 | 185 |

Referring to Table 2, when an additive according to the present invention was used, room temperature cycle-life characteristics were improved.

**Evaluation 2: Evaluation of High-temperature (45 °C) Cycle-life Characteristics**

The rechargeable lithium battery cells according to Examples 1 to 6 and Comparative Examples 1 to 6 were 200 cycles charged and discharged at 45 °C under 0.33 C charge (CC/CV, 4.3V, 0.025 C Cut-off) /1.0 C discharge (CC, 2.5 V Cut-off) conditions and then, measured with respect to capacity retention rates and DC-IR (direct current internal resistance) variation ratio.

Capacity retention and DC-IR was calculated according to Equations 1 and 2 based on a voltage changed by applying a current of SOC 50 C for 30 seconds, and the results are shown in Table 3.

(Table 3)

| | Capacity retention (@45°C, 200 Cycles) | Initial DC-IR @45°C (mΩ) | DC-IR after 200 cycles @45°C (mΩ) | DC-IR variation ratio after 200 cycles @45°C (%) |
|---|---|---|---|---|
| Example 1 | 95.5 | 40.4 | 71.4 | 177 |
| Example 2 | 92.1 | 44.4 | 81.3 | 183 |
| Example 3 | 89.5 | 42.7 | 76.9 | 180 |
| Example 4 | 91.9 | 40.2 | 98.5 | 245 |
| Example 5 | 95.4 | 40.7 | 73.2 | 180 |
| Example 6 | 86.5 | 42.6 | 110.7 | 260 |
| Comparative Example 1 | 41.2 | 51.4 | 175.4 | 341 |
| Comparative Example 2 | 59.1 | 46.6 | 86.7 | 186 |

(continued)

| | Capacity retention (@45°C, 200 Cycles) | Initial DC-IR @45°C (mΩ) | DC-IR after 200 cycles @45°C (mΩ) | DC-IR variation ratio after 200 cycles @45°C (%) |
|---|---|---|---|---|
| Comparative Example 3 | 77.1 | 46.2 | 89.4 | 194 |
| Comparative Example 4 | 83.7 | 43.3 | 86.0 | 199 |
| Comparative Example 5 | 68.5 | 47.2 | 86.5 | 182 |
| Comparative Example 6 | 70.1 | 45.9 | 84.9 | 185 |

Referring to Table 3, when an additive according to the present invention was used, high temperature cycle-life characteristics were improved.

**Evaluation 3: ICP-MS Analysis (Ni and Mn Elution Evaluation)**

The rechargeable lithium battery cells according to Examples 1 to 6 and Comparative Examples 1 to 6 were 200 cycles charged and discharged under 0.33 C charge (CC/CV, 4.3 V, 0.025 C Cut-off) /1.0 C discharge (CC, 2.5 V Cut-off) conditions at 25°C and then, measured with respect to Ni and Mn elution amounts in the following method.

The rechargeable lithium battery cells were disassembled to separate positive electrode plates therefrom. Subsequently, the separated positive electrode plates were put with an electrolyte in a 10 ml TEFLON (tetrafluoroethylene) container, which was sealed and measured with respect to Ni and Mn contents through an ICP-MS analysis, and the results are shown in Table 4. Meanwhile, the electrolyte was a solution in which 1.5 M $LiPF_6$ was dissolved in a mixed solvent of EC (ethylene carbonate), DEC (diethyl carbonate), and EMC (ethylmethyl carbonate) (a volume ratio of 2:1:7).

(Table 4)

| | Ni detection amount (ppm) | Mn detection amount (ppm) |
|---|---|---|
| Example 1 | 311 | 24 |
| Example 2 | 388 | 30 |
| Example 3 | 395 | 35 |
| Example 4 | 402 | 42 |
| Example 5 | 320 | 20 |
| Example 6 | 300 | 18 |
| Comparative Example 1 | 2241 | 278 |
| Comparative Example 2 | 1721 | 141 |
| Comparative Example 3 | 754 | 80 |
| Comparative Example 4 | 459 | 43 |
| Comparative Example 5 | 636 | 92 |
| Comparative Example 6 | 586 | 57 |

Referring to Table 4, the rechargeable lithium battery cells according to Examples 1 to 6 exhibited very low Ni and Mn elution amounts from the electrode plates. However, when the rechargeable lithium battery cells according to Comparative Examples 1 to 6 were compared with the rechargeable lithium battery cells of the examples, significantly large amounts of Ni and Mn were eluted. Accordingly, in the rechargeable lithium battery cells of the examples, an amount of gas generated as the cycles progressed was significantly reduced.

**Evaluation 4: Evaluation of High-temperature Storage Characteristics (Capacity Retention Rate/Capacity Recovery Rate/DC-IR Variation Ratio)**

The rechargeable lithium battery cells according to Examples 1 to 6 and Comparative Examples 1 to 6 were once charged and discharged at 0.2 C and measured with respect to charge and discharge capacity (before high temperature storage).

In addition, the rechargeable lithium battery cells according to Examples 1 to 6 and Comparative Examples 1 to 6 were charged in SOC 100% (a state in which charged to 100% of charge capacity, when total charge capacity was 100%), stored at 60 °C for 30 days, discharged to 3.0 V at 0.2 C under a constant current condition, and then, measured with respect to initial discharge capacity.

In other words, the cells were recharged to 4.3 V under a constant current condition of 0.2 C at a cut-off current of 0.05 C under a constant voltage condition and discharged to 3.0 V under a constant current condition of 0.2 C and then, twice measured with respect to discharge capacity. A ratio of first discharge capacity to the initial discharge capacity was obtained as capacity retention (retention capacity), and a ratio of second discharge capacity to the initial discharge capacity was obtained as a capacity recovery rate (recovery capacity).

The rechargeable lithium battery cells according to Examples 1 to 6 and Comparative Examples 1 to 6 were measured with respect to $\Delta V/\Delta I$ (voltage change/current change) as initial DC-IR, and then, DC-IR were measured again by making a maximum energy state inside the cells to a full-charge state (SOC 100%) and storing the cells at a high temperature (60 °C) for 30 days to calculate a DC-IR variation ratio (%) according to Equation 3, and the results are shown in Table 5.

[Equation 3]

$$\text{DC-IR variation ratio} = (\text{DC-IR after 30 days} / \text{initial DC-IR}) * 100$$

(Table 5)

| | Capacity retention (@60°C, 30D) | Capacity recovery rate (@60°C, 30D) | Initial DC-IR (@25°C, mΩ) | DC-IR @60°C, 30D (mΩ) | DC-IR variation ratio (%) |
|---|---|---|---|---|---|
| Example 1 | 97 | 99 | 41.2 | 57.3 | 139 |
| Example 2 | 94 | 96 | 47.5 | 68.9 | 145 |
| Example 3 | 91 | 95 | 43.8 | 65.7 | 150 |
| Example 4 | 75 | 83 | 40.9 | 109.8 | 271 |
| Example 5 | 96 | 99 | 41.4 | 60.0 | 145 |
| Example 6 | 92 | 98 | 42.5 | 70.2 | 164 |
| Comparative Example 1 | 33 | 52 | 42.0 | 215.1 | 511 |
| Comparative Example 2 | 52 | 62 | 43.6 | 100.7 | 231 |
| Comparative Example 3 | 60 | 75 | 44.6 | 97.7 | 219 |
| Comparative Example 4 | 88 | 90 | 43.3 | 69.7 | 161 |
| Comparative Example 5 | 77 | 80 | 45.4 | 84.4 | 186 |
| Comparative Example 6 | 72 | 78 | 47.2 | 83.5 | 177 |

Referring to Table 5, the rechargeable lithium battery cells according to Examples 1 to 6, compared with the rechargeable lithium battery cells according to Comparative Examples 1 to 6, exhibited improved capacity retention rates and capacity recovery rates during the high-temperature storage but were suppressed from resistance change rates.

**Evaluation 5: Measurement of Amount of Generated Gas after High-temperature Storage**

The rechargeable lithium battery cells according to Examples 1 to 6 and Comparative Examples 1 to 6 were charged to 4.3 V (vs. Li) at 0.1 C under a constant current condition and subsequently, cut off at 0.05 C under a constant voltage mode while maintaining 4.3 V at 25 °C. Subsequently, the rechargeable lithium battery cells were disassembled, and positive electrode plates therefrom were put with an electrolyte in a pouch and then, stored in a 60 °C oven for 30 days. A volume change due to a mass change of the pouch was converted in an Archimedes method, and the results are shown in Table 6. Meanwhile, the electrolyte was a solution in which 1.5 M $LiPF_6$ was dissolved in a mixed solvent of EC (ethylene carbonate), DEC (diethyl carbonate), and EMC (ethylmethyl carbonate) (a volume ratio of 2:1:7).

The Archimedes method was a method of measuring a weight of the pouch in a water tank filled with water at every specific period (e.g., 4 days) and converting the weight change into a volume change to measure an amount of generated gas.

(Table 6)

| | Amount of generated gas (mL) |
|---|---|
| Example 1 | 0.7 |
| Example 2 | 0.9 |
| Example 3 | 0.9 |
| Example 4 | 1.1 |
| Example 5 | 0.7 |
| Example 6 | 0.8 |
| Comparative Example 1 | 2.8 |
| Comparative Example 2 | 1.6 |
| Comparative Example 3 | 2.2 |
| Comparative Example 4 | 1.2 |
| Comparative Example 5 | 1.3 |
| Comparative Example 6 | 1.3 |

Referring to Table 6, the rechargeable lithium battery cells of Examples 1 to 6 exhibited a reduced amount of generated gas, compared with the cells of Comparative Examples 1 to 6.

Resultantly, the rechargeable lithium battery cells manufactured by using an electrolyte including an additive according to the present invention exhibited simultaneously improved room temperature cycle-life and resistance characteristics and also, cycle-life and resistance characteristics when left at a high temperature.

Accordingly, the rechargeable lithium battery cells according to the scope of the examples of the present invention exhibited improved electrolyte impregnation property and realized excellent cycle characteristics and also, reduced resistance after the high-temperature storage and thus improved high temperature stability.

<Description of Symbols>

100: rechargeable lithium battery

112: negative electrode

113: separator

114: positive electrode

120: battery case

140: sealing member

**Claims**

**1.** An additive for an electrolyte represented by Chemical Formula 1:

[Chemical Formula 1]

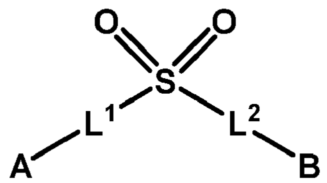

wherein, in Chemical Formula 1,

$L^1$ and $L^2$ are each independently a single bond, a substituted or unsubstituted C1 to C5 alkylene group, a substituted or unsubstituted C2 to C5 alkenylene group, a substituted or unsubstituted C2 to C5 alkynylene group, or a substituted or unsubstituted C6 to C20 arylene group,
A and B are each independently a substituted or unsubstituted C1 to C20 alkyl group, a substituted or unsubstituted C6 to C20 aryl group, or a substituted or unsubstituted C2 to C20 heteroaryl group, and
at least one of A and B is a group represented by Chemical Formula A,

[Chemical Formula A]

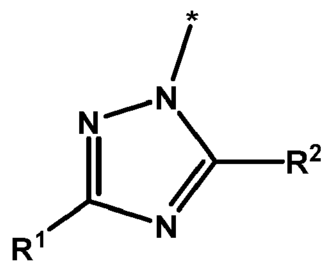

wherein, in Chemical Formula A,
$R^1$ and $R^2$ are each independently hydrogen, a halogen, a substituted or unsubstituted C1 to C10 alkyl group, or a substituted or unsubstituted C3 to C10 cycloalkyl group, wherein "substituted" refers to replacement of at least one hydrogen by deuterium, a halogen, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C10 fluoroalkyl group, or a cyano group.

**2.** The additive of claim 1, wherein
at least one of $L^1$ and $L^2$ is a substituted or unsubstituted C1 to C5 alkylene group.

**3.** The additive of claim 1, wherein
$L^1$ and $L^2$ are each independently a substituted or unsubstituted C1 to C5 alkylene group.

**4.** The additive of claim 1, wherein
at least one of $L^1$ and $L^2$ is a substituted or unsubstituted C2 to C5 alkylene group.

**5.** The additive of claim 1, wherein
$L^1$ and $L^2$ are each independently a substituted or unsubstituted C2 to C5 alkylene group.

**6.** The additive of claim 1, wherein
Chemical Formula 1 is represented by Chemical Formula 1-1:

[Chemical Formula 1-1]

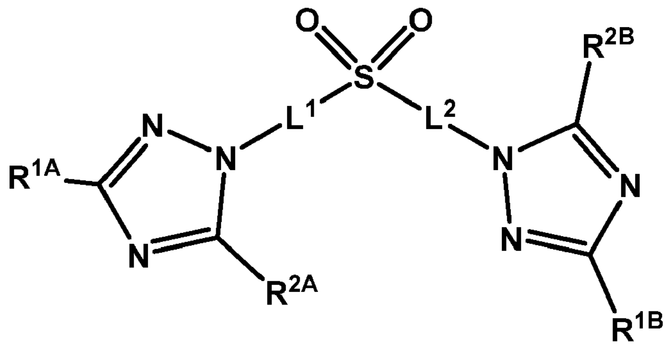

wherein, in Chemical Formula 1-1,

$L^1$ and $L^2$ are each independently a substituted or unsubstituted C1 to C5 alkylene group, and
$R^{1A}$, $R^{1B}$, $R^{2A}$ and $R^{2B}$ are each independently hydrogen, a halogen, a substituted or unsubstituted C1 to C10 alkyl group or a substituted or unsubstituted C3 to C10 cycloalkyl group.

**7.** The additive of claim 1, wherein
the additive is selected from the compounds of Formula 1a or Formula 1b:

[Formula 1a] 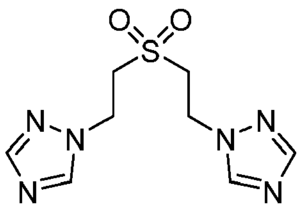 [Formula 1b] 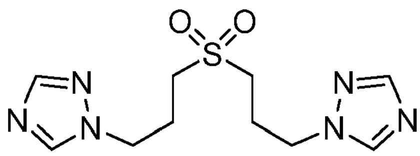 .

**8.** An electrolyte for a rechargeable lithium battery, comprising
a non-aqueous organic solvent, a lithium salt, and of the additive for the electrolyte of any one of the preceding claims.

**9.** The electrolyte of claim 8, wherein
the additive is included in an amount of 0.01 to 5.0 parts by weight, preferably 0.05 to 3.0 parts by weight, based on 100 parts by weight of the electrolyte for the rechargeable lithium battery.

**10.** The electrolyte of claim 8, wherein
the electrolyte further includes at least one other additive of vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene carbonate, vinylethylene carbonate (VEC), adiponitrile (AN), succinonitrile (SN), 1,3,6-hexane tricyanide (HTCN), propenesultone (PST), propanesultone (PS), lithium tetrafluoroborate ($LiBF_4$), lithium difluorophosphate ($LiPO_2F_2$), and 2-fluoro biphenyl (2-FBP).

**11.** A rechargeable lithium battery (100), comprising

a positive electrode (114) including a positive active material;
a negative electrode (112) including a negative active material; and
the electrolyte for the rechargeable lithium battery of any one of claim 8 to claim 10.

**12.** The rechargeable lithium battery of claim 11, wherein the negative active material includes at least one of graphite and a Si composite.

**13.** The rechargeable lithium battery of claim 12, wherein the Si composite includes a core including Si-based particles and an amorphous carbon coating layer.

**14.** The rechargeable lithium battery of claim 13, wherein the Si-based particles include at least one of Si particles, a Si-C composite, $SiO_x$ ($0 < x \leq 2$), and a Si alloy.

**15.** Use of a compound represented by Chemical Formula 1:

[Chemical Formula 1]

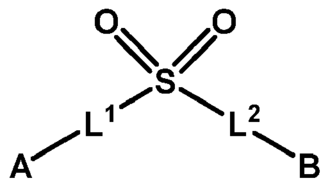

wherein, in Chemical Formula 1,

$L^1$ and $L^2$ are each independently a single bond, a substituted or unsubstituted C1 to C5 alkylene group, a substituted or unsubstituted C2 to C5 alkenylene group, a substituted or unsubstituted C2 to C5 alkynylene group, or a substituted or unsubstituted C6 to C20 arylene group,
A and B are each independently a substituted or unsubstituted C1 to C20 alkyl group, a substituted or unsubstituted C6 to C20 aryl group, or a substituted or unsubstituted C2 to C20 heteroaryl group, and
at least one of A and B is a group represented by Chemical Formula A,

[Chemical Formula A]

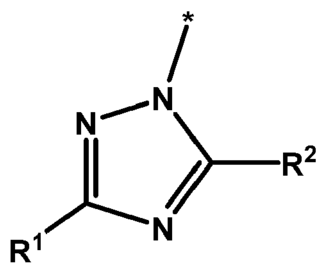

wherein, in Chemical Formula A,
$R^1$ and $R^2$ are each independently hydrogen, a halogen, a substituted or unsubstituted C1 to C10 alkyl group, or a substituted or unsubstituted C3 to C10 cycloalkyl group as an additive for an electrolyte of rechargeable lithium battery.

【FIG. 1】

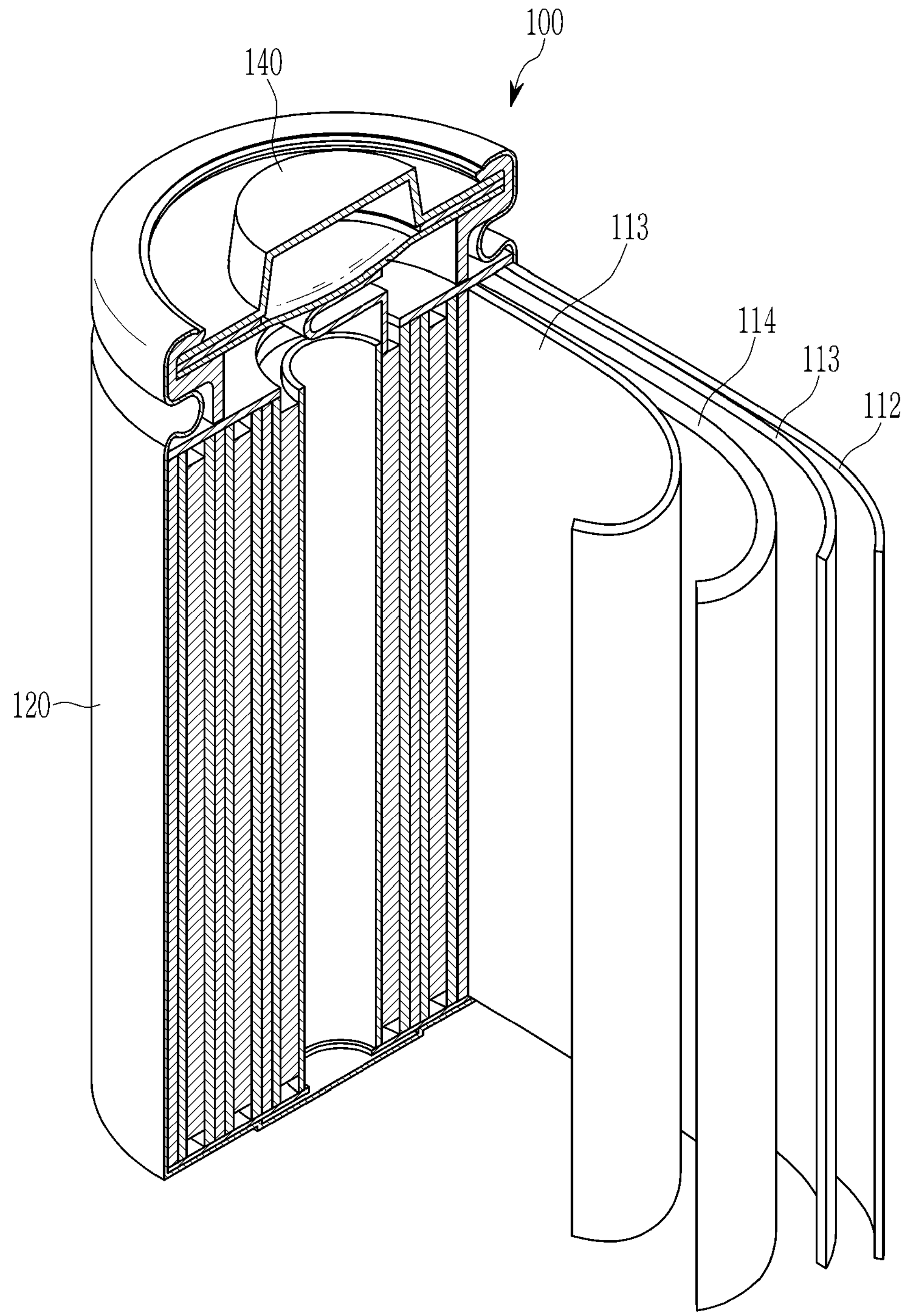

Europäisches Patentamt
European Patent Office
Office européen des brevets

# EUROPEAN SEARCH REPORT

Application Number
EP 23 18 7725

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2021/009529 A1 (JOHNSON MARTIN REID [US]) 14 January 2021 (2021-01-14)<br>* claims 1,10,11,22 *<br>* figure 1 * | 1-7<br>8-15 | INV.<br>C07D249/08<br>C07D403/02<br>H01M10/0566 |
| A | US 2013/298386 A1 (TARASCON JEAN-MARIE [FR] ET AL) 14 November 2013 (2013-11-14)<br>* claim 6 * | 1-15 | |

TECHNICAL FIELDS SEARCHED (IPC)
C07D
H01M

The present search report has been drawn up for all claims

| Place of search | Date of completion of the search | Examiner |
|---|---|---|
| The Hague | 9 January 2024 | Domínguez Gutiérrez |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO.

EP 23 18 7725

This annex lists the patent family members relating to the patent documents cited in the above-mentioned European search report. The members are as contained in the European Patent Office EDP file on
The European Patent Office is in no way liable for these particulars which are merely given for the purpose of information.

09-01-2024

| Patent document cited in search report | Publication date | Patent family member(s) | Publication date |
|---|---|---|---|
| US 2021009529 A1 | 14-01-2021 | NONE | |
| US 2013298386 A1 | 14-11-2013 | CN 103038924 A | 10-04-2013 |
| | | EP 2583337 A1 | 24-04-2013 |
| | | FR 2961634 A1 | 23-12-2011 |
| | | JP 6097686 B2 | 15-03-2017 |
| | | JP 2013529830 A | 22-07-2013 |
| | | KR 20130076859 A | 08-07-2013 |
| | | US 2013298386 A1 | 14-11-2013 |
| | | WO 2011157958 A1 | 22-12-2011 |

EPO FORM P0459

For more details about this annex : see Official Journal of the European Patent Office, No. 12/82